## Europäisches Patentamt
## European Patent Office
## Office européen des brevets

(19)

(11) Publication number: **0 152 517**
**B1**

(12)

# EUROPEAN PATENT SPECIFICATION

(45) Date of publication of patent specification: **02.11.88**

(51) Int. Cl.⁴: **A 61 J 3/07**

(21) Application number: **84109170.5**

(22) Date of filing: **02.08.84**

(54) Apparatus and method for sealing capsules.

(30) Priority: **22.02.84 US 582364**

(43) Date of publication of application:
**28.08.85 Bulletin 85/35**

(45) Publication of the grant of the patent:
**02.11.88 Bulletin 88/44**

(84) Designated Contracting States:
**AT BE CH DE FR GB IT LI LU NL SE**

(56) References cited:
EP-A-0 116 743
DE-B-1 217 024
GB-A- 956 300
US-A-2 738 827
US-A-2 924 920
US-A-3 071 513
US-A-3 073 087
US-A-3 159 546
US-A-3 538 677

(73) Proprietor: **WARNER-LAMBERT COMPANY**
**201 Tabor Road**
**Morris Plains New Jersey 07950 (US)**

(72) Inventor: **Wittwer, Fritz**
**Im Budler 15**
**CH-4411 Lupsingen (CH)**

(74) Representative: **Silbiger, Jakob, Dr.**
**c/o CAPSUGEL AG Münchensteinerstrasse 41**
**CH-4002 Basel (CH)**

Courier Press, Leamington Spa, England.

# 0 152 517

**Description**

This invention relates to methods for sealing capsules, using melting point depression mixtures and thermal energy; and apparatus for sealing such capsules.

The capsules sealed by utilizing the present invention are telescopically joined capsules, having coaxial cap and body parts. The capsules are made of gelatin whose properties are pharmaceutically acceptable.

In this application, when the term 'gelatin' is used, it is also understood to include other proteins similar to gelatin in physical and chemical properties.

In addition, capsules are sealed having a cap and/or body part made from a gelatin foam as disclosed in applicant's European application, application No. 83304741.8, the disclosure of which is incorporated herein by reference. Foam capsules contain a microdispersion of a gas in a gelatin wall. The capsule body and cap portions are formed by dip-molding the film-forming mixture obtained by a microdispersion of the gas in a gelatin solution; optionally with the inclusion of a plasticizer and/or coloring agent, and/or flavoring agent, and/or foam stabilizer, and/or gelatin extender.

By a suitable choice of the gas proportion in the capsule wall and its micronization level, it is possible, within certain limits, to control the capsule wall disintegration speed and its opacity.

Hard shell gelatin capsules have a disadvantage when compared with other dosage forms, in that the cap and the body parts can be opened and rejoined without the disruption becoming externally visible or tamper-evident. Therefore, the consumer has no real guarantee that the contents of a capsule have not been tampered with.

Telescopically joined capsules have only a partial overlap of the cap side wall over the body side wall which allows gripping and withdrawal of the body, thereby making separation relatively easy. The present invention uses melting point depression mixtures containing water in combination with thermal energy applied to the overlap of the cap side wall over the body side wall to completely seal the overlap of the capsule parts. In addition, when completely sealed, the capsules can be used for liquids without the problems of potential leakage.

Prior art for capsule sealing is contained in the following patents:

US Patent No. 3,071,513, discloses a sealing fluid comprising a dispersion of an air-drying hydrophilic, film-forming polymer in an organic solvent. The application of the sealing fluid is by dipping the capsules.

US Patent No. 3,159,546, discloses a liquid sealant consisting of three components containing by weight from about 1 to 4 1/2 parts, preferably 3 to 4 1/2 parts, of acetone; from about 1 1/2 to 2 parts, and preferably 1 1/4 to 2 parts, of water; and from about 3/4 to 2 1/4 parts, and preferably about 3/4 of a part, of ethyl acetate. The application of the liquid solvent was by drop application.

US Patent No. 2,924,920, discloses a three components mixture containing a polyhydric alcohol, an alcohol and water. This composition is used to seal capsules by a swelling technique. The process is designed to avoid solvent penetrating the overlap between capsule body and cap.

French Patent No. 2,118,883, discloses the use of alcohol and water in an enteric coating process.

It has now been found that if a melting point depression mixture containing water is applied to the overlap of the cap and body parts of the capsules, e.g. by dipping the capsules in such a mixture, and after removing the melting point depression mixture from the exposed capsule surface, heat of 40°C or higher is applied to the capsules, and excellent liquid proof seal of the overlap of the capsules is obtained without any deformation of the capsules and without any formation of blisters or change of the surface, e.g. its lustre.

This invention refers to a method of sealing a hard shell gelatin capsules having coaxial cap and body parts which overlap when telescopically joined, the method comprising contacting the edge of the cap part of the capsule with a melting point depression sealing fluid to cause said fluid to be evenly distributed between the overlapping sections of the body and cap part and removing the excess sealing fluid from the exposed capsule surface of said capsule characterized in that

(a) the sealing fluid comprises from about 20% to about 98% of an aliphatic monohydric alcohol having one to four carbon atoms which may be substituted by one alkoxy group having one or two carbon atoms or mixtures thereof and from about 2% to about 80% of water; and

(b) whilst said sealing fluid is still present within the region of overlap thermal energy is applied to locally melt the contacting surfaces of the overlapping sections of the body and cap parts enabling a complete fusion together of said overlapping sections, wherein processes (i) to (iv)

(i) 75% ethanol/25% water as sealing fluid, and heat applied by hot air at 70°C;

(ii) 60% ethanol/40% water as sealing fluid, and heat applied by hot air at 70°C;

(iii) 60% ethanol/40% water as sealing fluid, and heat applied by infra-red heating means at 60°C;

(iv) 60% ethanol/40% water as sealant fluid, and heat applied by infra-red heating means at about 80°C, are disclaimed.

Our European Patent Application No. 83 30 53 30.9 discloses a method for sealing capsules having hard shell coaxial cap and body parts which overlap when telescopically joined, comprising the steps of dipping the capsules in a sealing fluid making contact by capillary action within the overlap of the cap and body parts; removing the sealing fluid from the surface of the capsules; and applying thermal energy to the overlap so as to cause a sealing together of the cap and body parts.

That Application No. 83 30 53 30.9 discloses the use of a wide range of sealing fluids, including a few

2

**0 152 517**

specific lower ethanol and water mixtures, as well as a few specific temperatures for the application of thermal energy to capsules which have had such specific lower ethanol and water mixtures applied to the region of overlap.

In view of these specific disclosures in Application No. 80 30 53 30.9, processes (i) to (iv), as defined above, are disclaimed.

The present invention is concerned with applying heat at the sealing stage b) in a temperature range from 40 to 170°C, with the exception of the concentrations 75% ethanol and 25% water, and 60% ethanol and 40% water, for the temperatures of 60°C and 70—140°C.

The present invention is particularly, but not exclusively, concerned with those cases in which the sealing temperature is 40 to 68°C with the exception of 60°C, preferably in the range from 40 to 65°C with the exception of 60°C, more preferably 42 to 65°C with the exception of 60°C, preferably using heated air.

As organic solvent or mixtures of solvents mentioned in step a) above, compounds having a maximum of six carbon atoms and especially aliphatic monohydric alcohols having one to four carbon atoms which may be substituted by one alkoxy group having one or two carbon atoms or mixtures thereof are preferred but also other organic solvents or their mixtures which are miscible with water like a mixture of acetone and ethylacetate may be used.

In step a) mentioned above, the edge of the cap part of the capsule is briefly contacted, under 20 seconds, preferably under 5 seconds, and most preferably under 1 second, with the melting point depression mixture which is instantaneously evenly distributed between the overlapping sections of the capsule body and cap parts by means of capillary forces but also wets the exposed outside surface of the capsule.

The melting point depression mixture is preferably used in liquid form but can also be introduced from its vapor phase by suitable condensation.

The capsules may simply be immersed in the liquid melting point depression mixture or contacted, using a fluid jet system which will deliver measured quantities of the mixture in a high frequency pulsating manner directly at the cap edge of the capsule. Other methods may be used such as spraying, contacting with solid materials impregnated or wetted with the melting point depression mixture, continuous wave bonding, etc.

It should be noted that the contacting time is generally uncritical as the distribution of the melting-point depression mixture within the overlapping section is nearly instantaneous, i.e. in much less than one second.

In a further step, the melting point depression mixture is removed by one or more of a number of draining and drying procedures from the exposed outside surface of the capsule. Initially draining is followed by air drying. Draining may be performed by the use of agitation, vibration, impact or air flow. Due to capillary forces, the denaturation-melting point depression mixture remains in the gap between the overlapping sections of the capsule body and cap parts. The mixture is now specifically where it is needed. While time is not critical, from above zero to about 6 minutes, preferably from above 0 to about 3 minutes would be employed to remove the melting point depression mixture.

In step b), complete sealing of the capsules which are impossible to separate without mutilation, is accomplished in a third step. The addition of a controlled quantity of thermal energy to the overlapping sections of body and cap parts then gives a liquid proof seal. The thermal energy may be applied through convection such as hot air flow, conduction such as by applying a hot metal stamp or bar, by electromagnetic irradiation such as microwaves or infra-red heat. It is assumed that the application of heat causes the melting of gelatin to occur prior to the evaporation of the melting point depression mixture from within the gap between the overlapping sections of the capsule body and cap parts, thus giving this strong seal.

Where convection and infra-red energies are employed, a time from about 1 to 15 minutes, preferably 2 to 6 minutes, is generally used.

Where conduction energy is derived from a hot metal stamp or bar at a temperature of approx. 100° to 170°C, a time of about 0.1 to 5 seconds, preferably 0.5 to 3 seconds, is generally used.

Where microwaves are employed, a time of from about 1 to about 5 seconds, preferably 1.5 to 3 seconds, is generally used.

While not preferred, drying of step b) and step c) may be combined into a single operation which is also intended to be an embodiment of the present invention.

The melting point depression mixture is evenly distributed between the overlapping section of the cap and body parts of the gelatin capsule by capillary effect. This effect is achieved when the contact angle between a drop of the melting-point depression mixture and the gelatin film is small.

The wettability of gelatin films is measured as 'adhesional wetting' where a liquid not originally in contact with a substrate makes contact with that substrate and adheres to it.

The contact angles between gelatin films and solvents were measured by use of a microscope fitted with a goniometer eyepiece.

The tests were performed on a gelatin film whereby the contact angle was measured 20 seconds after depositing a drop of a liquid on the gelatin film. The following Table I shows the measured contact angles. From this table, one readily observes the high wetting effect achieved by adding alcohol to water which is directly related to the highly observed capillary effect.

3

TABLE 1

| Sealing fluids | Mean contact angles |
|---|---|
| water | 83° +/− 6° |
| 75% aqueous ethyl alcohol | 3.5° +/−1° |
| 90% aqueous methanol solution (not detectable) | near to 0° |

The alcohols of the melting point depression mixtures that may be employed in this invention are aliphatic monohydric alcohols of from 1 to 4 carbon atoms which may also be substituted by one alkoxy group having one or two carbon atoms, and mixtures thereof.

Of prime importance is the miscibility of the alcohols with water and their ease of removal after contact with the capsules. In view of this, the preferred alcohols are n-propanol, iso-propanol, ethanol, methanol and mixtures thereof. In light of toxicity and safety concerns, the most preferred is ethanol. The alcohols used in the present invention are employed in combination with water. The relative ratios of the alcohol to water must be in ranges so that all the components are completely miscible in each other.

The preferred ratios of alcohol to water are dependent on a number of factors:
1. Composition of the gelatin
    a. types of gelatines and relative proportions thereof
    b. molecular weight distribution
    c. water content
    d. dyes and pigments
2. Markings on the capsule surface.
3. Temperature used for the heat-sealing phase.

In the process, according to the present invention, it is assumed that the alcohol denaturates the gelatin while the water lowers the melting point of the gelatin and promotes its swelling. The foregoing results in a contact of the locally denatured and molten gelatin surfaces of the overlapping sections of capsule cap and body parts, enabling a complete fusion together of said overlapping sections by applying the thermal energy during the sealing step.

The percents of organic solvents, preferably alcohol as defined above, and water are based on a ratio of organic solvent respectively alcohol to total solution on a volume/volume base. The percent organic solvent resp. alcohol which may be one or a mixture of the alcohols of the invention, is from about 20% to about 98% while that of water is from about 2% to about 80%; preferably, the organic solvent resp. alcohol which may be one or a mixture of the alcohols of the invention is in a range of 30% to 95%, the water content being 5 to 70%. The most preferred range is one wherein the organic solvent resp. alcohol concentration is 45% to 93% while that of water is 7 to 55%.

Higher concentrations of organic solvents will generally be used with lower molecular weight gelatins or gelatin blends, gelatins with lower bloom values, gelatins having a high water content, higher sealing temperatures or high water-soluble dye content of the gelatin.

Higher concentrations of water will generally be used with higher molecular weight gelatins or gelatin blends, gelatins with higher bloom values, gelatins having a low water content, lower sealing temperatures, high pigment content of the gelatin or with capsules having alcohol sensitive printing on the surface.

One may incorporate other components such as surfactants to further promote the capillary effect or other ingredients that would not interfere with the melting point depression mixture. However, surfactants are generally not necessary to achieve the desired results of this invention.

In step a) of the process according to the present invention, the temperature of the denaturation melting point depression mixture is preferably from 5 to 40°C, more preferably from 10 to 25°C, the most preferred range being from 15 to 20°C.

The removing of the excess liquid from the exposed outside surface of the capsule is preferably performed at a temperature of from 10 to 35°C, more preferably from 20 to 35°C, the most preferred being in a range from 25 to 32°C.

Step b) of the process uses heat in a temperature range of from about 40°C to about 170°C, preferably about 40°C to about 68°C, the most preferred being 40°C to 65°C, and in particular as mentioned above in a temperature range of 42°—65°C each with exception of 60°C using heated air.

In general, the suitable temperature range will be 40—55°C for an alcohol concentration of up to 60% and higher than 55°C for alcohol concentrations of more than 60% using hot air, e.g. in a fluidized bed.

When the higher temperatures are employed, one generally uses the conduction energy derived from a hot metal stamp or bar.

Intermediate ranges are used when employing infra-red thermal energy while temperatures in the lowest range are employed with convection energy involving hot air.

When a microwave source is relied upon, the electromagnetic irradiation found to be most effective was at frequencies of about 2.4 GHz for an exposure of about 1 to 5 seconds, preferably 1.5 to 3 seconds, with a strength of field in the range of 200 V/cm. It was observed that microwaves of this strength of field and time caused efficient melting of the material within the overlapping sections of cap and body parts and resulted in gelatination of the material so as to make a strong physical bond or seal therein.

It was also noted that the use of microwaves at such levels does not deform the capsules if the exposed outside surface of the capsules is quickly dried according to this invention.

Of the numerous heating processes, the process relying upon hot air is the most preferred.

Any suitable type of gelatin may be used for the capsules to be treated according to the present invention, such as gelatines prepared from pigskin, calfskin or bones, fabricated by acid or lime treatment and blends of various types of gelatines. Preferred are gelatines with a bloom gel strength as gelatin wall of the capsules of above 120 g, preferably from 150 g to 300 g, the most preferred range being from 180 g to 240 g, measured according to the method described in 'Method of Analysis A.O.A.C., 13th Edition 1980, chapter 23, page 374'.

In addition to the selection of temperatures to be used being based on the source of heat, other factors also should be considered in deciding upon a temperature when practicing this invention. More specifically increased water content in the melting point depression mixture requires the use of lower temperatures. In addition, the presence of pigments in the capsule wall calls for the use of higher temperatures during the sealing process.

The sealing of capsules by the present invention can be used for gelatin capsules which have been telescopically joined and have the following contents:

a. Empty;
b. Powder;
c. Pastes;
d. Tablets, pellets, granules, microcapsules, etc.
e. Liquids (the sealing of the present invention was also successful in preventing leakage of oil from within the gelatin capsule);
f. Liquids in solids or the reverse; and
g. Any combination of contents b)—f).

With the process one not only obtains a tamper-proof capsule but also a hermetically sealed liquid-proof capsule ideally suited for liquids, pastes, creamy substances, oxygen-sensitive materials and moisture vapor sensitive materials.

For the sealing of gelatin capsules filled with oils, it was noted that an inverse capillary effect driving the oil between the overlapping sections of the body and cap parts of the gelatin capsules may occur, especially when the filled gelatin capsules are held in a cap part down position. For rape seed oil, having a viscosity of above about 90 centipoises, a large contact angle between the gelatin film and the oil was measured which means that the capillary forces of oil are much lower than the capillary forces of the sealing fluids. Therefore, if the gelatin capsules are sealed within a few minutes after filling with an oil, the oily capsule content does not enter between the overlapping sections of the body and the cap parts of the gelatin capsule. Hence, the capsules can be sealed by the sealing fluids of the present invention.

If liquids or oils with low viscosities below about 90 centipoises and small contact angles are used, the following measures accomplished a complete, hermetic sealing by the present invention:

— sealing the gelatin capsules within a few seconds after ejection from the filling machine;
— holding the gelatin capsule in an upright position with the cap part on top during the sealing process;
— cooling the liquid contents prior to filling into the gelatin capsule in order to increase the viscosity and the contact angle between the gelatin film and the liquid;
— adding a thickness agent to the liquid contents prior to the filling process.

Description of the apparatus for sealing capsules

The present invention relates also to an apparatus for sealing hard shell gelatin capsules having coaxial cap and body parts which overlap when telescopically joined, characterized in that it has the following three working stations:

(A) a station in which at least the edge of each cap part is contacted with the melting point depression sealing fluid,

(B) a station for removing the mixture from the exposed capsule surface,

(C) a fluidized bed device to heat seal the overlapping sections of the cap and body parts of said capsules by effecting melting of the contacting surfaces.

In such an apparatus according to the present invention, the station in which the excess of mixture is removed can consist of a draining-off device connected with a subsequent drying device.

Ideally, the contacting station will include a tank for the mixture and a motor-driven cylindrical or conical rotatably-mounted wire-mesh basket, one end of which is immersed in the mixture whereby the basket contains an internal helix for drawing the capsules out of the mixture. The portion of the basket not immersed in the mixture can thus work as a draining-off device. This draining-off device can expediently be combined with the outlet of a blower and eventually also with the inlet of a suction device. The draining-off device may also be designed otherwise, e.g. as a vibrating conveyor with vertical arranged nails which contact the capsules only with their tips. The draining-off device may then be connected to a further basket designed as a subsequent drier. The contacting station defined in the invention can also be designed otherwise: it may, for example, be fitted with a rotatably mounted paddle-wheel, partially immersed in a container holding the mixture and enclosed in a cylindrical housing. provided with openings for the mixture, the diameter of which and axial length are identical to those of the paddle-wheel whereby an inlet and an outlet opening are located above the level of the mixture.

In the scope of the present invention there are also various possibilities for the design of the heating station: thus one may, for example, use a cylindrical basket rotatably mounted on an approximately horizontal axis fitted with at least one closable opening and having equipment to fill it and heat it and having also equipment to empty it once the contacting surfaces of the overlapping sections of capsule's body and cap parts have been sufficiently melted together. A further design of the invention provides as a drying device and as a heating station two fluidized-bed dryers whereby each dryer is connected with the draining-off device or the contacting station respectively and is provided with a control circuit in such a way that in operating stage A one dryer works as a drying device and the other as a heating station while in operating stage B the two fluidized-bed dryers switch over their functions so that the capsules to be processed travel to either one of the fluidized-bed dryers or to the other to receive successively both drying and heating treatments there.

In the following, a few examples of apparatus according to the invention are described in connection with the drawings. The drawings show:

In Fig. 1: Large-scale, a commercial, filled, closed capsule containing a pharmaceutical product in the form of a powder or granulate;

In Fig. 2: A capsule sealed in accordance with the invention, likewise large-scale;

In Fig. 3: A schematic side view of a first design of an apparatus in accordance with the invention for the complete sealing of the capsules;

In Fig. 4: A schematic side view of a second design of an apparatus according to the invention;

In Fig. 5: A detail of an alternate design of the apparatus of Fig. 4;

In Fig. 6: A detail of another alternate design of the apparatus of Fig. 4;

In Fig. 7: A schematic side view of a third design of an apparatus according to the invention;

In Fig. 8: A simplified top view of a fourth design of an apparatus according to the invention;

In Fig. 9: A section along the line IX—IX of Fig. 8;

In Fig. 10: A side view of a further design for a contacting station;

In Fig. 11: A schematic view of a further design of a station for contacting the cap edge.

In Fig. 12+13: A schematic view of two further embodiments of contacting stations. Further embodiments are shown by Figs. 14—27.

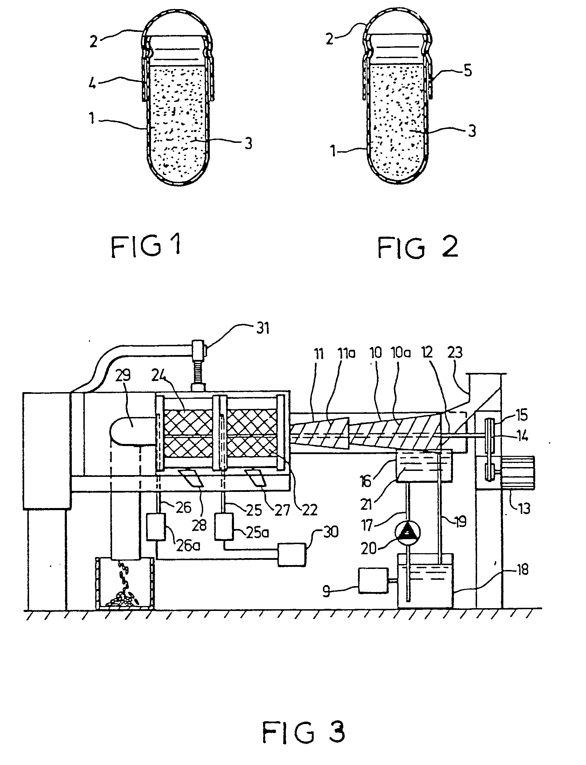

In Fig. 1 is shown a closed gelatin capsule having coaxial body part 1 and cap part 2 which overlap when telescopically joined, and filled with a pharmaceutical drug formulation 3, here in the form of a powder or granulate. This kind of capsule is commercially available, but in this form cannot be filled with liquids since any liquid would leak through the cylindrical gap 4 between body and cap as soon as the capsule would not be stored in a vertical position.

Fig. 2 shows a similar capsule in which, however, the body part 1 and the cap part 2 are strongly and tightly bonded by an effective seal 5 according to the invention, in such a manner that the capsule parts cannot be separated without mutilation and so that the capsule is also tight against leakage of liquid contents.

Sealing of gelatin capsules may be performed with the apparatus described as follows and only schematically shown in Fig. 3. Two conical wire-mesh baskets, one marked 10 and the other 11, are connected with each other, incapable of independent rotation and rotatably mounted by means of a shaft 12. A motor 13 is connected via a belt 14 to a pulley 15, fitted on the shaft 12, and serves to keep the two baskets revolving at a constant speed. The end of the basket 10 near the motor is immersed in a tank 16, holding the gelatin melting point depression mixture 21. By means of a feed tube 17, fed by the pump 20, and of an overflow tube 19 ending in a second tank 18, one ensures that the level of the mixture 21 in tank 16 is kept constant so that the basket 10 is always immersed to the same depth in the mixture. By means of a device designated 9, it is ensured that the concentration of the mixture remains constant. Since the mixture consists e.g. of alcohol and water, the device 9 may consist for example of the following components: an alcohol tank connected via a dosing pump with the tank 18, a density-meter fitted on-line on one of the tubes 17 or 19, and a control device which, when the density-meter indicates an excessive density, sets the feed pump in action for a short time. In each of the baskets there is a screw 10a or 11a respectively to transport the capsules from the mixture 21, into which they are introduced via a funnel marked 23, to the basket 11 and thence to the connected, also rotatably mounted and motor-driven, cylindrical wire-mesh basket 22 which at one end has an opening for the inflow and at the other end an opening for the outflow of the capsules. With basket 22 there is connected a wire-mesh basket 24 likewise rotatably mounted and motor-driven and also provided at both ends with openings for the inflow and outflow respectively of capsules. In this basket, the two openings are closable by means of a programmer 30 controlling the closure devices 25 or 26 respectively, both activating organs being marked 25a and 26a in Fig. 3. An air outlet 27 belonging to basket 22 is designed in such a manner that the outcoming air can blow-over the capsules from basket 22 into the basket 24 while an air-outlet 28 belonging to basket 24 is

# 0 152 517

designed so that the outcoming air can blow-over the capsules from basket 24 into the exit tube 29. Said air-outlets 27 and 28, shown in Fig. 3 for simplification reasons in a vertical position below the baskets 22 and 24 respectively, are preferably mounted horizontally beside said baskets. Belonging to basket 24, there is also the outlet of a hot air blower 31 to maintain the interior of the basket at the desired and needed temperature. Said hot air is preferably blown from below the basket and approximately tangentially to it, in the same direction as the basket rotates. This apparatus now works as follows: The filled and closed capsules, preferably immediately after they have been closed, move as far as possible directly via the inlet funnel 23 to the mixture in the part of basket 10 immersed in the tank 16 which works as contacting station. By means of the screw 10a in the basket 10 the capsules are drawn after a predetermined period out of the mixture 21 whereby the period is determined by the depth of immersion of the basket 10 in the mixture 21 and by the revolving speed of this basket. During this period, the mixture penetrates by capillary action between the overlapping sections of the body and cap parts of the capsules. This period is to be chosen in such a manner that the gap between the overlapping sections of capsule's body and cap parts are filled to a certain extent without however the mixture penetrating to the interior of the capsule. The time needed depends on the dimensions of the capsules and on the viscosity and the surface tension of the mixture and may lie e.g. in the range of 0.1 second to 5 seconds. It can be determined from case to case by spot checks. The connecting part of the basket 10 which is not in the mixture and the basket 11 then serve as a draining-off device in which the mixture, still wetting the exposed outer surface of the body and the cap parts, largely drains-off, thus avoiding denaturation of the surface of the gelatin and reduction of the melting point there. The draining-off device may of course be provided with means for supplying cold or warm air and also with means for withdrawing by suction such air that may contain droplets of the mixture. In the connected wire-mesh rotating basket 22 occurs a subsequent drying of the capsule's exposed outside surfaces which is best performed by means of a blast of dry air, and ensures that said exposed outside surfaces are dried before the mixture has the possibility to promote their swelling and/or denaturation and modify their physical properties. The programmer 30 then causes the opening of the closure device 25 via the operating organ 25a and then the starting of an air blast from the outlet 27 lasting as long as most of the capsules have been blown-over from the basket 22 into the basket 24, working as heating station, whereupon the closure device 25 is reshut. After the necessary time for the local melting of the contacting surfaces of the overlapping sections of the body part 1 and the cap part 2, resulting in the sealing of the capsules (which time depends on the composition of the capsule wall material, the size of the capsule, the width of the gap between the sections of body and cap parts of the capsule, the composition of the mixture as well as on the temperature in the basket 24 which may be reached by introduction of heated air or by direct heating such as for example by infrared heating and which must be determined on a case to case basis), the closure device 26 is opened via the operating organ 26a and then starts an air blast from the outlet 28 lasting as long as all the capsules, now completely sealed, have been blown over from the basket 24 into the outlet 29 of the apparatus. When once the closure device 26 is shut, the closure device 25 is re-opened and the next batch of outside dried capsules is blown into the basket 24 whereupon the process described above is repeated. In another embodiment of the invention, the thermal energy is applied by irradiating the capsules in basket 24 with a micro-waves source such as to selectively heat the contacting overlapping sections of body and cap parts of the capsules where the mixture is located.

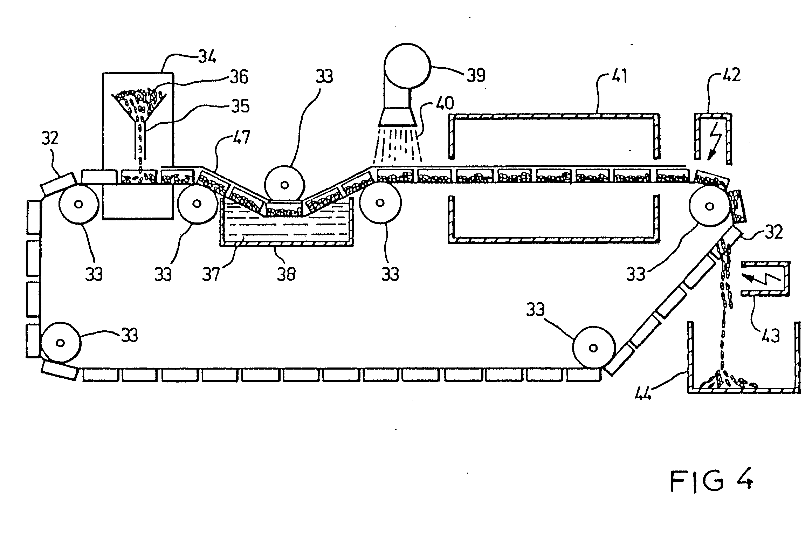

Fig. 4 shows a second possibility for the design of an apparatus in accordance with the invention. Forty to fifty containers 32 of perforated or wire-mesh material are connected into an endless chain conveyed over seven pulleys 33. At a loading station 34 the containers are loaded via a funnel 35 with capsules 36. Thence they go to a contacting station which comprises a tank 38 containing the mixture 37. This mixture has the same properties as that designated by 21 in the first example described in connection with Fig. 3 and therefore exerts the same action. The contacting time with the mixture may be adjusted either by varying the guidance of the endless chain or by adapting the conveying speed of said endless chain, such as the mixture is allowed to penetrate and penetrates into the gap between the overlapping sections of cap and body parts of the capsule but not into the capsule's inside. It is clear that certain measures must be taken to ensure that all the capsules in a container must be sufficiently contacted; this may be done e.g. by providing the containers with a cover or by conveying the containers through below a perforated or wire-mesh cover plate 47. When the capsules have been withdrawn from the mixture, they are conveyed through a blast of air 40 blown from a fan 39 located above or below the containers 32 in order to remove the excess mixture from the exposed outside surface of the capsules.

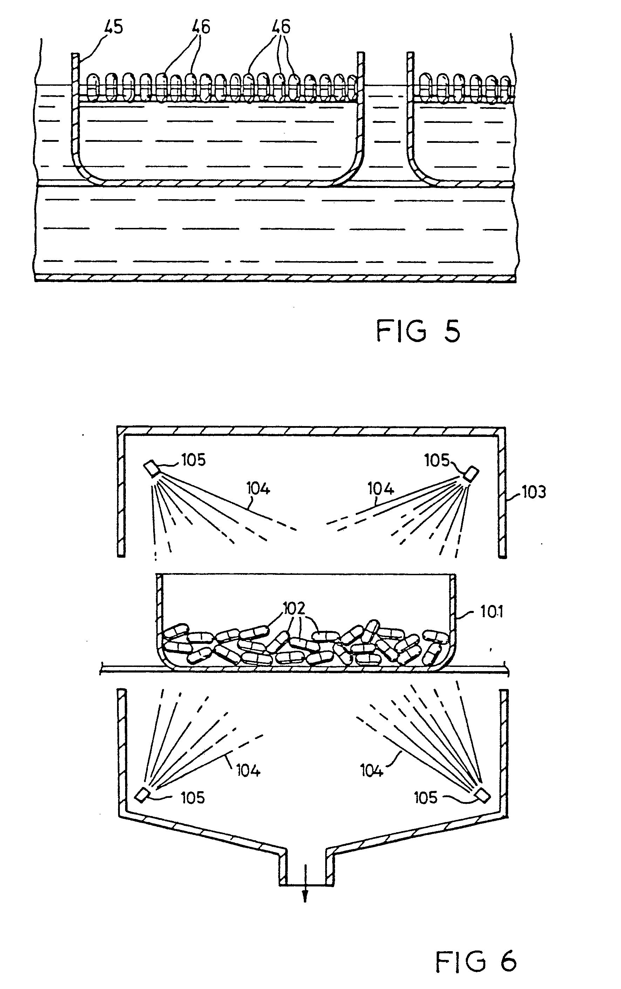

Thereafter, they are conveyed through a drying chamber 41 where they are outside dried. A perforated or wire-mesh cover-plate 47 is also provided to prevent blowing-away of the capsules 36 during processing beneath fan 39 and through drying chamber 41. Subsequent heating of the capsules may take place also in the drying chamber 41, preferably by means of hot air or infrared heating, or beneath an energy source 42 which may be e.g. a micro-wave source which only heats the wet zones between the overlapping sections of body and cap parts to the temperature required to seal the capsules. Where the containers 32 are emptied, there may be alternate or additional energy sources 42 and/or 43 which form a heating station. Finally, the sealed capsules are collected in a container 44 for further processing and shipment. Whereas on the apparatus schematically shown in Fig. 4 the capsules are conveyed randomly in the containers 32, Fig. 5 shows an alternative type of containers 45 wherein the filled and telescopically joined capsules 46 are oriented and held in an upright cap up position, e.g. by means of plates provided with holes or other

7

holders so that it is possible to contact with the mixture only the exposed outside surfaces of the bodies and the lower edges of the caps but not the residual outside surface of the caps, in other words so that the body edges are constantly held above the level of the mixture. This arrangement is designed especially for capsules containing liquids, so as to avoid that the capsule's contents may penetrate between the overlapping sections of body and cap. In these containers 45, the capsules drain off better under the influence of the blower thus increasing the hourly output.

Fig. 6 shows another alternative of a contacting station of the apparatus shown in Fig. 4 wherein the containers 101 containing the filled and telescopically joined capsules 102, instead of being immersed into a tank containing the mixture, are conveyed through a spray chamber 103 wherein mixture 104 is sprayed by nozzles 105 so as to contact the mixture 104 with at least the cap's edge of the capsule 102.

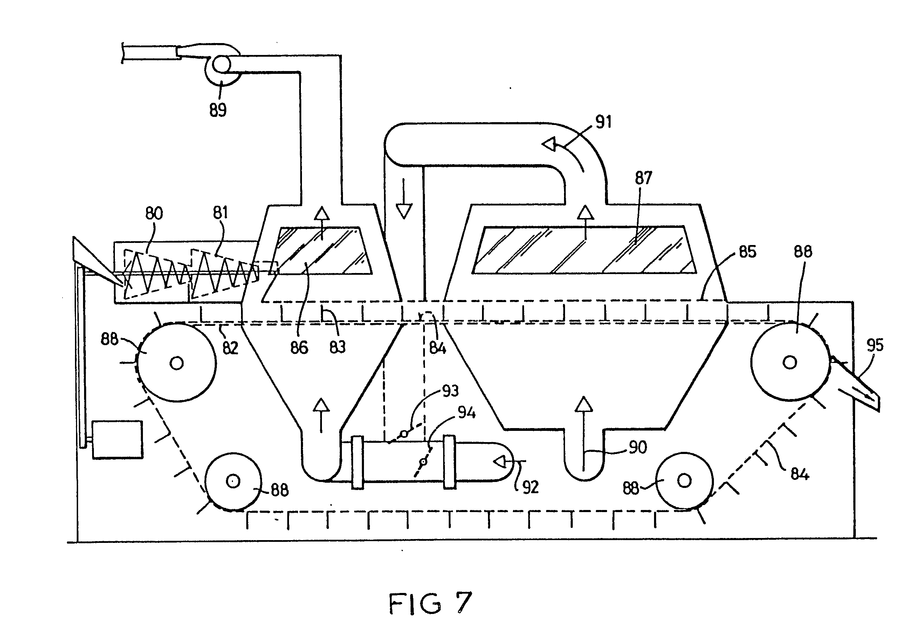

In a further alternative of the apparatus shown in Fig. 4, the loading station for the containers 32 or 45 is not located before the contacting station but only after the draining-off device so that the single containers are conveyed only through the drying device and the heating station. The contacting station and the draining-off device can be designed, for example, as described in connection with Fig. 3 or in accordance with the examples described in the following. A design according to this alternative is particularly of advantage when the time during which the capsules are in the contacting station is very short compared with the time during which they are in the other stations. One may also use pushers rather than containers 32 or 45 that can carry the capsules on a perforated band or in a groove or in a tunnel through the apparatus. Fig. 7 shows an apparatus according to such an alternative of the apparatus shown in Fig. 4. The contacting station 80 and the draining-off device 81 are identical to those composed of parts 10 to 20 described in connection with Fig. 3. From the draining-off device 81 the capsules fall continuously onto a perforated plate 82 with lateral walls. Pushers 83 mounted in equidistance on two motor-driven endless chains 84 are carried through a tunnel formed by the perforated bottomplate 82, its side walls and an upper perforated or wire-mesh cover 85. Said pushers 83 have the same dimensions as the square section of the tunnel and carry discrete quantities of capsules first through a drying device 86 where air is blown onto the capsules from below through the perforated plate 82. Afterwards said pushers carry them through a heating station 87 where hot air is blown onto the capsules, from below, through the perforated plate 83. The endless chains 84 are guided by four pulleys 88. The air flows in device 86 and in the station 87 may be obtained for examples by means of an exhaust fan 89. The air 90 may be heated up by any suitable device. The air 91 exhausted from the heating station 87 may be mixed with fresh air 92 and introduced into the drying device 86. Its temperature may be adjusted by the ratio between the two air-flows 91 and 92. This may be achieved for example by means of variable valves 93 and 94. Sealed capsules are discharged at the outlet 95. Contacting station and draining-off device may not only be restricted to those described in connection with Fig. 3. Any other suitable contacting station and draining off device may be used.

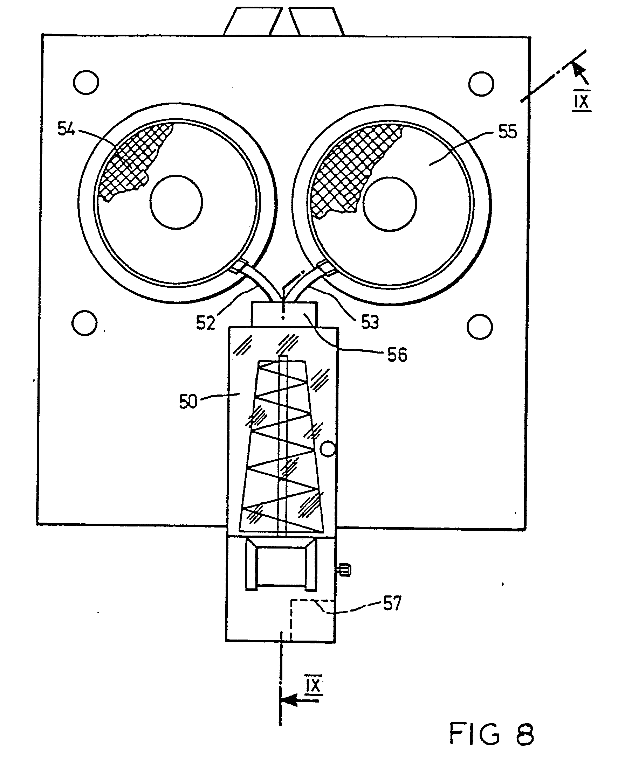

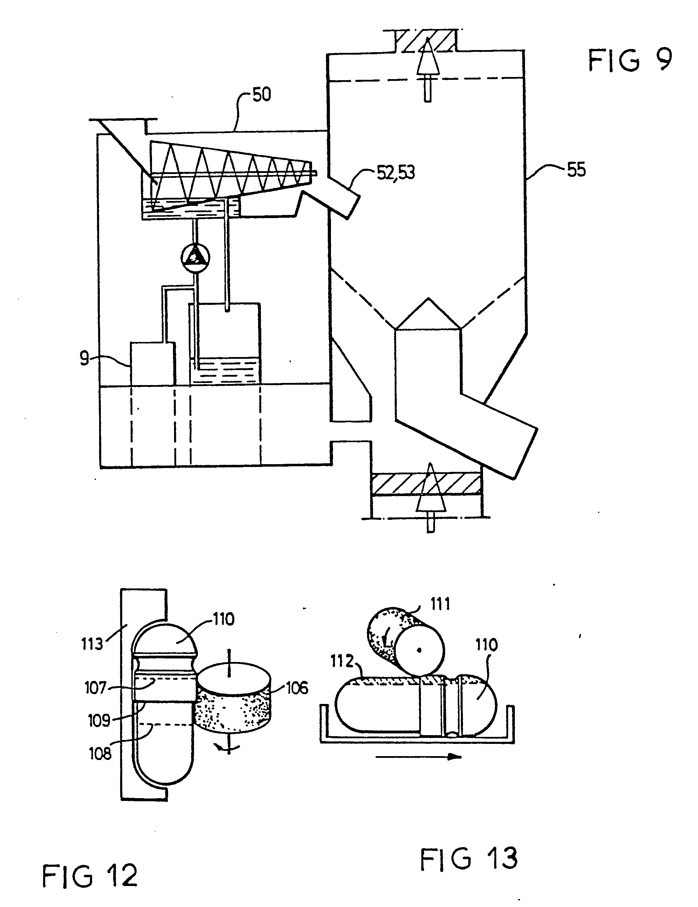

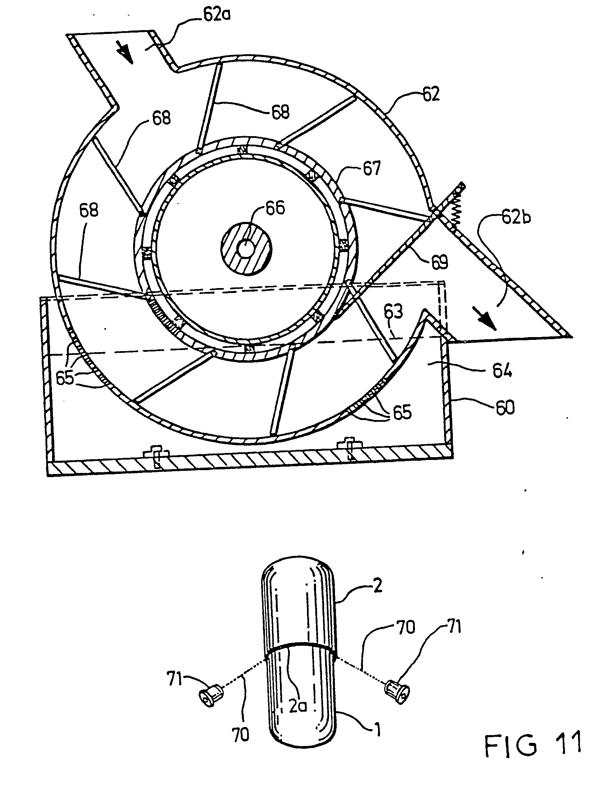

The apparatus shown in Fig. 8 and 9 comprises a combined contacting station and draining-off device 50 that is equivalent to those shown in Fig. 3, although this embodiment has only one basket. The draining-off device may however be designed otherwise, for example as a vibrating conveyor with vertically arranged nails and with means for the derivation of the drained-off mixture. From the draining-off device one duct 52 leads to a first fluidized bed dryer 54 and another duct 53 leads to a second fluidized bed dryer 55. At the entrance of both ducts there is a guiding unit 56 with a programmer-controlled guiding flap to direct alternatively the drained-off capsules during an adjustable time to the first fluidized bed dryer 54 or to the second fluidized bed dryer 55. A programmer 57 then acts so that in working condition A of the apparatus the capsules are continuously conveyed from the draining-off device to the first fluidized bed dryer 54 which works as a drying device while during the same time the second fluidized bed dryer 55 works first for a short time as a drying device, then during a longer time as a heating station and finally is emptied, and then, in working condition B of the apparatus, the capsules are continuously conveyed from the draining-off device to the second fluidized bed dryer 55 which works now as a drying device while during the same time the first fluidized bed dryer 54 works first for a short time as a drying device, then during a longer time as a heating station and finally is emptied, whereupon the programmer brings the apparatus back into the working condition A.

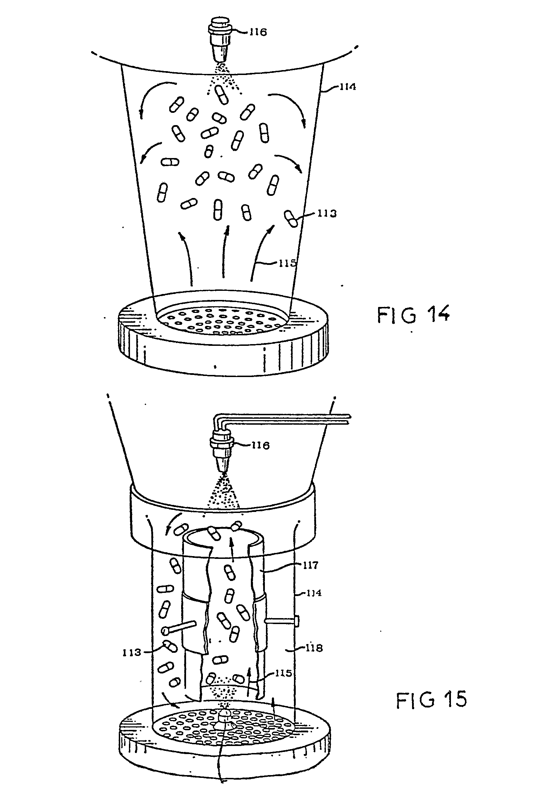

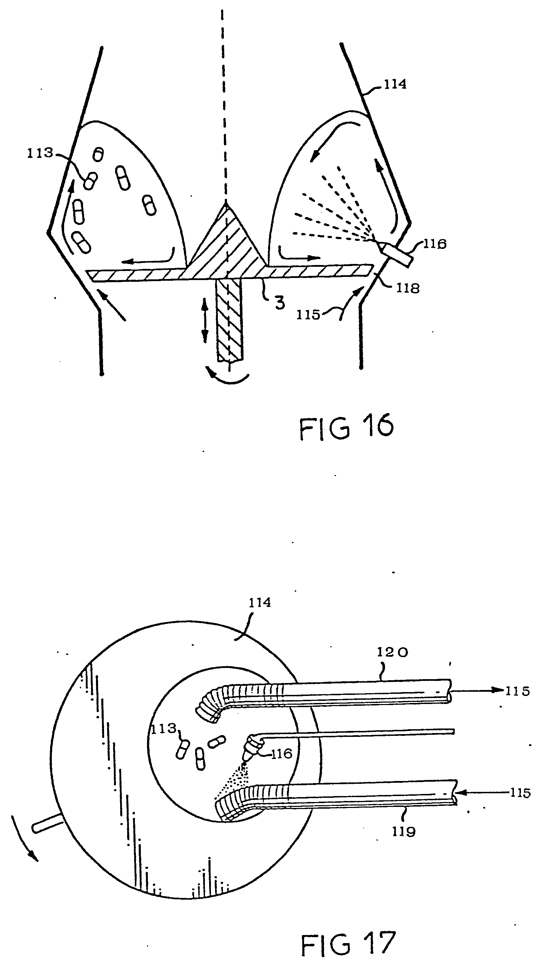

For this step certain conventional granulating or film coating equipment can be used. The sealing fluid is sprayed onto the capsules from above (Fig. 14) or from below (Fig. 15) or from various directions (Figs. 16 and 17). The quantity of sealing fluid may be controlled by a programmator and may be continuously or intermittently sprayed onto the capsules so that the water contained in the sealing fluid does not swell and deform the capsules or cause sticking together of the capsules.

Fig. 14 shows capsules 113 in a fluidized bed apparatus 114 wherein the capsules are moved by air 115 from below the capsules. The sealing fluid is introduced through a spray nozzle 116 from above.

Fig. 15 shows capsules 113 in a Wurster type film coating apparatus 114 wherein the capsules are moved by air 115 from below. A large air flow is circulated upward through an inner channel 117 wherein the capsules are conveyed upward, and outwardly and downwardly through an outer channel 118. The sealing fluid can be sprayed through either or both a spraying nozzle 116 located above or below the capsule.

Fig. 16 shows capsules 113 in a rotary fluidized bed granulator 114 wherein the capsules are moved both by rotation and by air flow 115 through a variable peripheral slit 118. The sealing fluid is introduced through a nozzle 116 at the side of the granulator.

8

# 0 152 517

Fig. 17 shows a perspective view of a coating pan 114 wherein capsules 113 are moved by rotation of the coating pan 114. Air flow 115 is through conduits 119, 120 into and out of this coating pan. A nozzle 116 introduces sealing fluid onto the capsules 113.

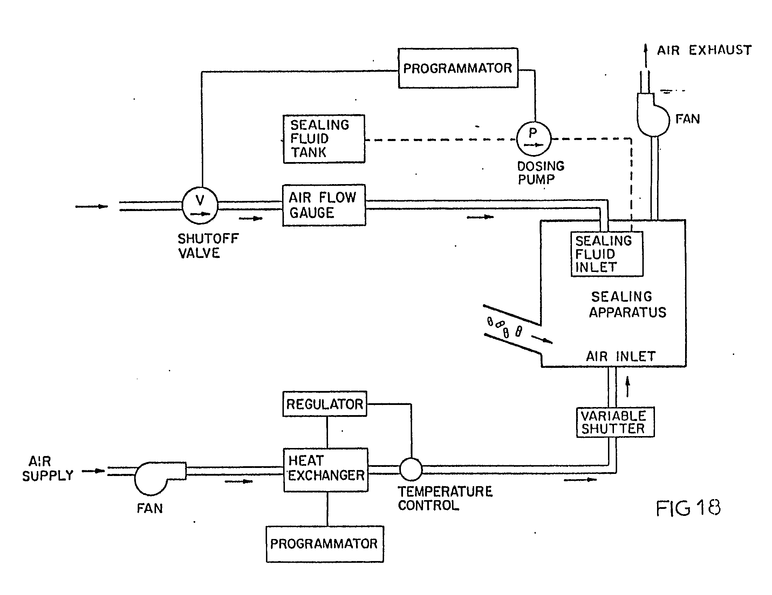

A further schematic in Fig. 18 is representative for the methods of the present invention as accomplished on conventional coating and granulating equipment which are generally available within most pharmaceutical facilities.

Fig. 10 shows a further design of a contacting station: A rotatably mounted paddle-wheel 61 with a horizontal axis dips partly into a tank 60 containing the mixture. Said paddle-wheel is mounted within a housing 62 whose diameter and axial length are identical to the diameter and axial length of the paddle-wheel.

The housing 62 has an inlet opening 62a and an outlet opening 62b, both located above the level 63 of the mixture 64. The housing is also provided with many openings 65 for the mixture 64 that ensure a good circulation of the mixture throughout the housing. The housing may also be made from wire-mesh or a sieve or a perforated metal sheet. The paddle-wheel has a carrier ring 67 for the paddles 68 that is connected rigidly with the shaft 66 and immersed in the mixture 64, the paddles here being formed as combs that comb with the stationary striping comb 69 fixed elastically to the housing 62. The above described apparatus works now as follows: The capsules entering the inlet opening 62a drop onto a paddle 68 and, as the paddle-wheel is rotated, fall into the mixture where the following paddle causes their immersion and their subsequent withdrawal. Because of the incline of the paddles, most of the capsules by themselves glide from the paddle towards the outlet opening 62b. Those which however for any reason remain stuck are striped-off by the striping-comb 69 and thus drop into the outlet opening whence they pass to a draining-off device. The necessary time spent in the mixture may be adjusted by selecting the revolving speed of the paddle-wheel.

A further possibility for the design of a contacting station is shown in Fig. 11. The body part 1 and the cap part 2 of the capsule are subjected in the region of the edge 2a of the cap 2 to the impingement of one, two or more brief jets 70 of the mixture. These jets 70 are delivered in metered quantities and in a high frequency pulsating manner by the nozzles 71. Such nozzles 71 with the ancillary pressure-producing and control means find application in the so-called ink-jet writers of the printers of computers. They may be used as such for the purpose of this invention if the control programme is adapted to the present purpose i.e. is so arranged that then when a capsule is positioned in the centre between two or more jetting nozzles, the necessary amount of mixture is jetted so that it can be distributed by capillary action within the gap between body and cap overlapping sections. This contacting station may be connected with any of the drying devices and heating stations described in the foregoing.

Still a further possibility for the design of a contacting station is shown in Fig. 12 and 13. The capsule 110 shown in Fig. 12 is guided by means of a capsule holder 113 which is a part of a continuous chain of capsule holders and which conveys the capsule through a contacting station where the capsule is contacted radially with at least one rotating wetting roll 106 so as to precisely wet the cap and body parts immediately adjacent to the cap edge 109 between lines 107 and 108 with the mixture, either all around the capsule or at least on a circumference section thereof. Thereby the mixture is allowed to penetrate between cap part and body part overlapping sections by means of capillary forces. Fig. 13 shows an alternate embodiment where the capsule 110 is guided by means of a capsule holder 114 which is a part of a continuous chain of capsule holders and which conveys the capsule through a contacting station where the capsule is axially contacted by a wetting roll 111 on at least a longitudinal strip 112. This kind of contacting station, involving only a partial contact of the capsule wall with the mixture, may be of interest for minimizing possible effects of the mixture on the aspect and the physical properties of the capsule wall. The rolls may preferably be made from spongious materials, including for example natural or synthetic sponges, or sponge-like polymeric foams, or felt. In a preferred alternative embodiment they may be continuously wetted with the mixture by means of a tube which may be in line with a pump, or by a mixture transfer from a primary roll rotating within a tank containing the mixture. Said chain comprising capsule holders according to Fig. 12 and 13 may of course be conveyed after the contacting station through a mixture removing station and a heating station. Said heating station may comprise at least one heated metal roll or wheel, the axis of which is parallel to the capsule's axis. This wheel or roll rotates against the outside of the cap part and transfers in this manner the thermal energy requested for the sealing to the overlapping capsule parts' section on one or more circumferential areas which may appear at the outside of the cap part or of sealed capsules as grooves.

It was also found that two-piece gelatin capsules treated with the sealing fluid are hermetically sealed by contacting with hot metal devices such as stamps, jaws, wires or wheels resulting in one or more circumferential by continuous seals, appearing at the outside of the overlap as one or more indented rings or bands. The metal devices exert just enough pressure and are heated to the optimum temperature so that both capsule parts are hermetically sealed together at the overlap of the cap and body part. The metal heating devices can be coated with materials to avoid sticking to the capsules.

When a water containing liquid is used, it is a further improvement to add a gelatin softener to the liquid so as to further decrease the brittleness of the seal.

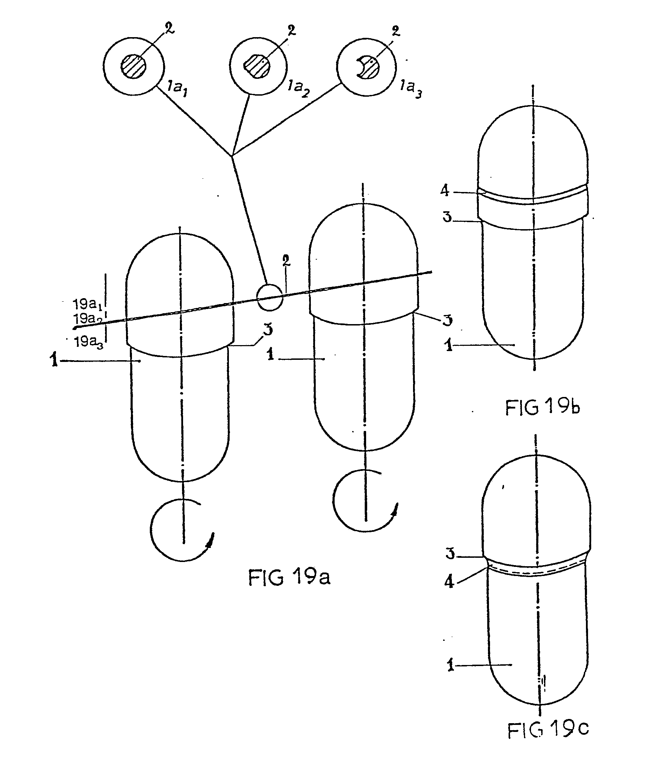

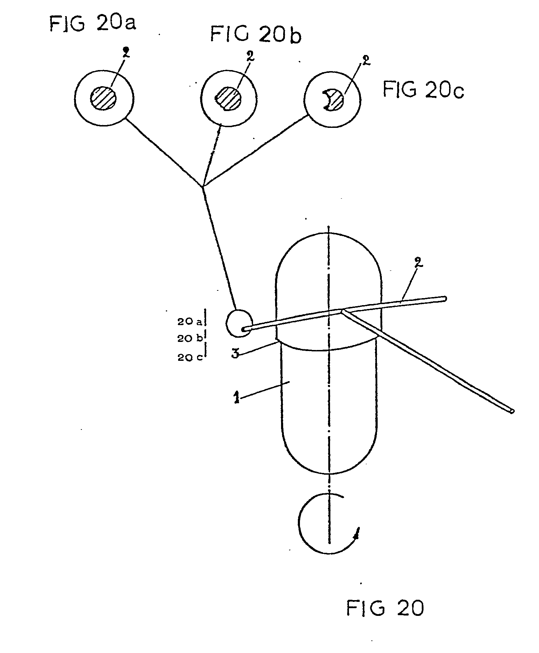

Figures 19 and 20 show the hermetic sealing of two-piece capsules, in accordance with the present invention, by rotating the filled and joined capsules at least 360° and simultaneously pressing the capsules

9

1 at the overlap against a hot wire or stamp 2 for a defined period of time. The sealing will appear at the outside of the overlap as a continuous circumferential ring 4 as shown in Fig. 19b. More than one ring may be applied to assure tightness. The sealing may be accomplished by applying the hot wire exactly at the outside seam of the overlap as shown in Fig. 19c. The wire may be adapted, as shown for example in Figs. 19a₁ to 19a₃ and 20a to 20c, in order to obtain special shapes, depths or breadths of the seal 4.

Alternatively, the capsules 1 could be fixed and the hot metal stamp 2 could rotate while contacting the cap side wall at the overlap. In order to obtain tightly sealed capsules, it is important to assure contacting the cap side wall at the overlap by a rotation of at least 360°. More than one wire may be applied around the cap side wall at the overlap in order to reduce the rotation angle.

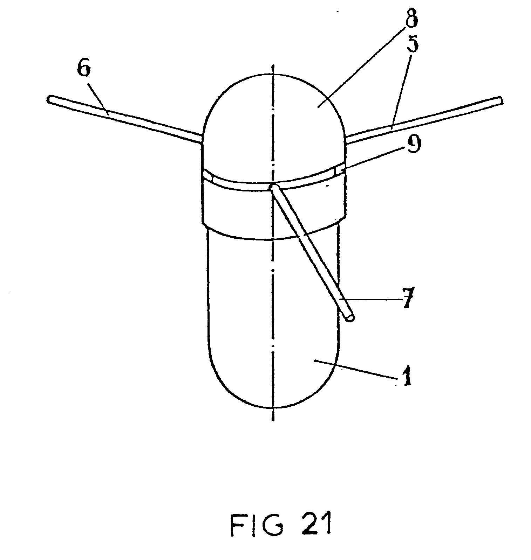

Fig. 21 shows a continuous circumferential sealing by contacting three hot-metal stamps 5, 6, 7 against the outside of the cap side wall 8 at the overlap. In order to obtain tightly sealed capsules, it is important to adjust the form and the dimensions of the stamps 5, 6, 7 so as to enclose the capsule 1 at the overlap in a continuous ring 9. The existence of small gaps between the stamps 5, 6, 7 during sealing are not critical because the local melting of gelatin is in a larger zone than the area contacted with each stamp.

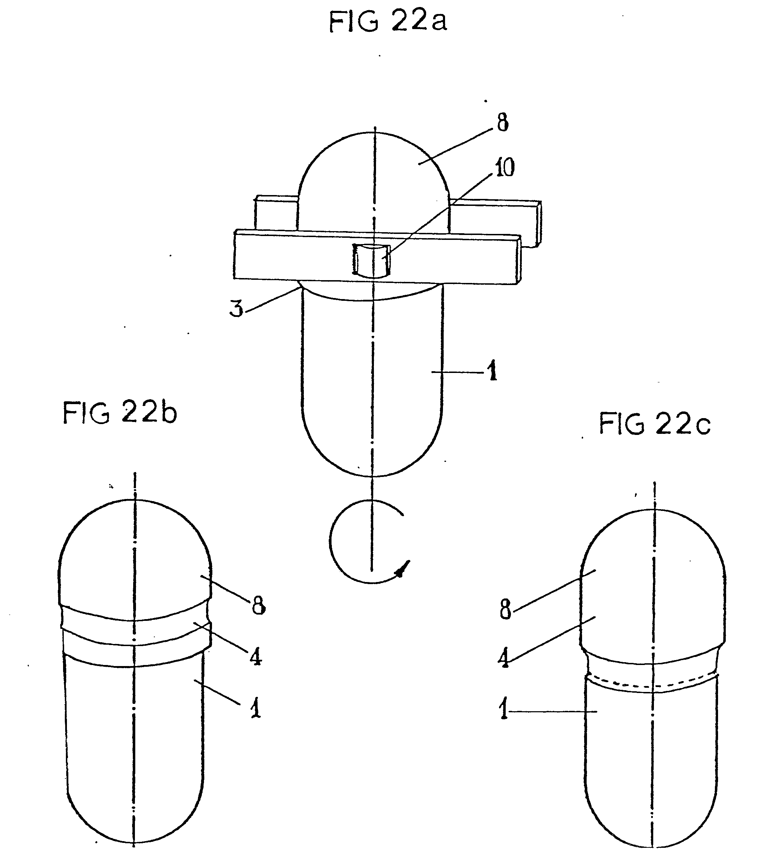

Fig. 22a illustrates sealing of the capsules by application of one or more hot metal wheels 10. The sealing 4 may be accomplished on the outside of the cap side wall 8 at the overlap (Fig. 22b) or on the seam 3 formed by the cap and body overlap (Fig. 22c).

A 360° seal is obtained either by rotating the capsule 1 or by rotating the wheels 10.

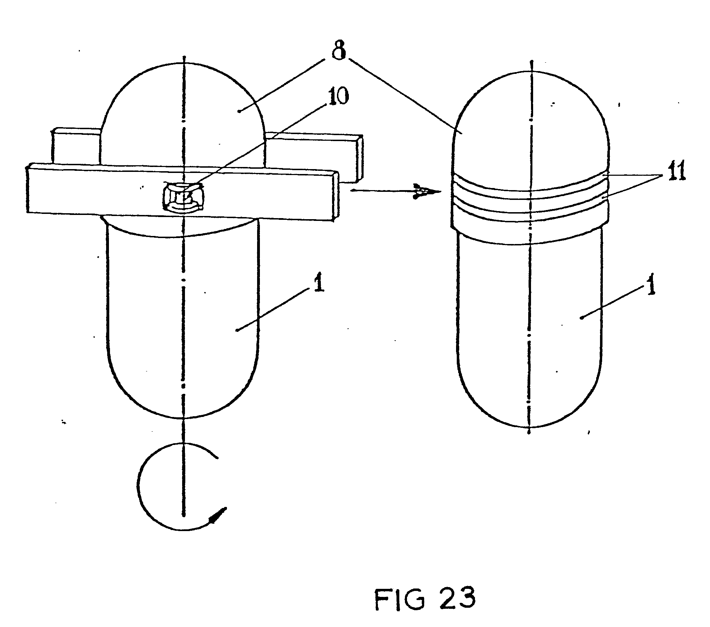

In Fig. 23 the seal 11, shown in Fig. 22, is doubled, thereby improving the tightness of the seal.

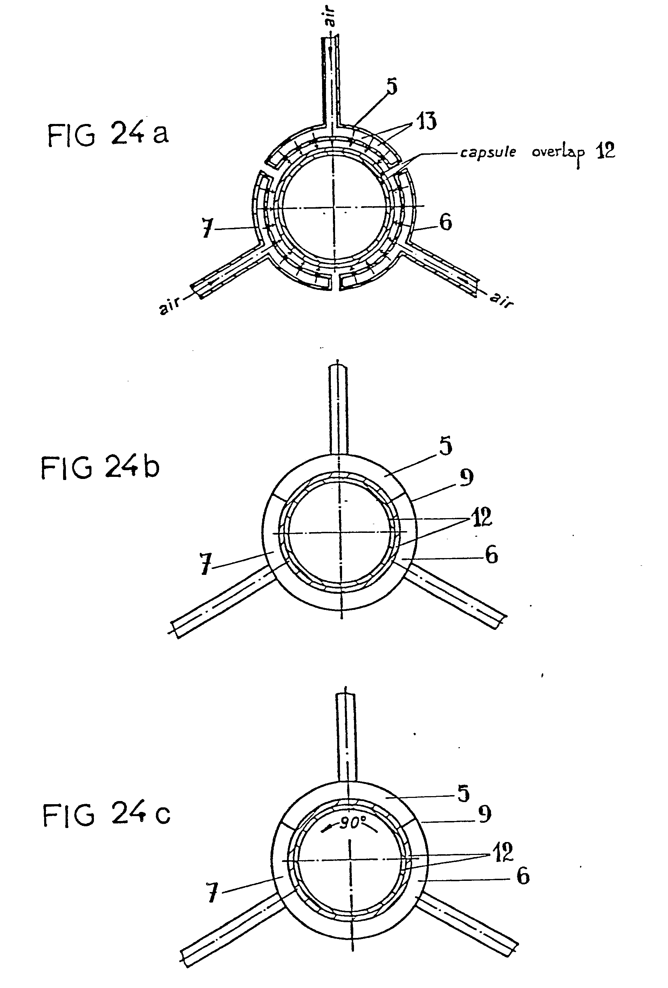

Fig. 24a illustrates another embodiment of the present invention. As in Fig. 21, the capsules are hermetically sealed by contacting three hot metal stamps 5, 6 and 7 against the outside of the cap side wall, so as to form a continuous ring 9 around the capsule (Fig. 24b). Before the application of the stamps, hot and wet air or normal water vapor can be blown into and/or onto the overlap 12 through small holes 13 fitted on the stamps (Fig. 24a). This increases the water content of the side walls and decreases the melting point of the gelatin therein as follows.

Simultaneously, the capsule side walls can be pre-heated. This allows use of stamps heated at lower temperatures, thus avoiding thermal degradation of the gelatin and of heat-sensitive capsule contents. Fig. 24c shows an optional additional rotation of 90° of the capsule 12 during application of the hot stamps 5, 6 and 7, thereby improving the seal tightness.

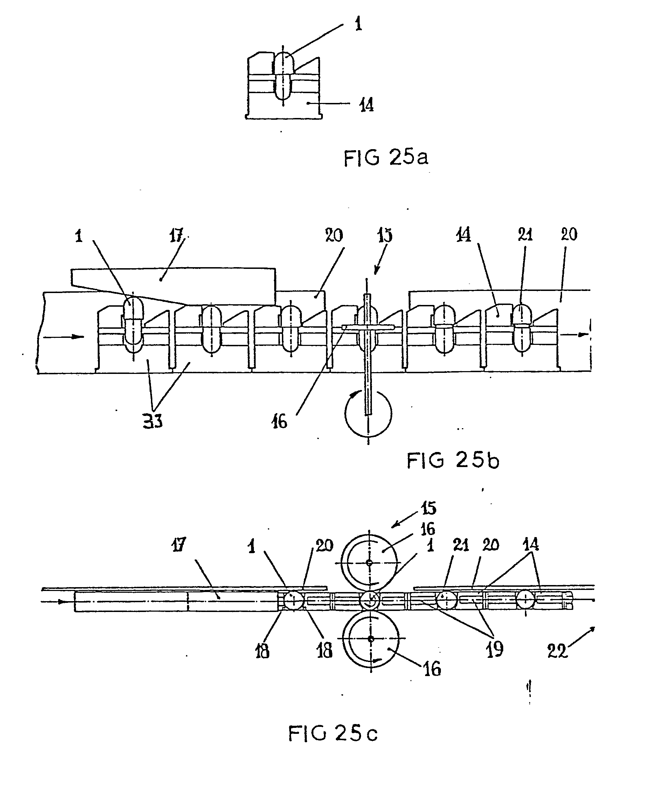

Fig. 25 shows the capsules being sealed on a special sealing apparatus, either connected to or separate from a capsule filling machine. The apparatus is a modified two-piece capsules radial imprinting machine: Hartnett Delta supplied by R. W. Hartnett Company, Philadelphia, Pennsylvania, USA.

This special sealing apparatus consists of four parts:

1. A *capsule rectification device* (not shown in Fig. 25) which delivers the joined capsules selectively in an axially cap-up position. This rectification is needed if the seal must be located on the cap part of the capsule, as shown in Fig. 18b, in order to avoid random application of the heated sealing devices to the body, thereby resulting in an unsealed capsule. If the seal must be located on the outside seam of the overlap (as shown in Fig. 18c), rectification may be needed to uniformly seal the capsules.

2. A *continuous chain 33*, having single capsule holders 14, which receives the filled, joined capsules 1 and conveys them individually through a sealing area 15. A lateral view of a single holder 14, containing a capsule 1 is shown in Fig. 25a.

3. A *sealing device* having two heated rolls 16 through which the capsules 1 are individually conveyed. The distance between the two rolls 16 is adjusted in order to exert the required circumferential pressure onto the capsules 1 to assure a hermetic seal and to avoid capsule damage. The capsules 1 rotate at least half a rotation during their passage through the rolls 16, thereby achieving a 360° sealing. One or more rolls can be used in order to obtain a continuous hermetic seal.

4. A sealed capsule *collecting system* (not shown). Fig. 25b shows a side view of the sealing area 15 and of a portion of the continuous chain 33. Fig. 25c is a top view of Fig. 25b. Capsules 1 are introduced into the single links 14, by a positioner 17 onto the continuous chain 32. The capsules 1 are maintained in the links 14 on one side by two curvatures 18 of the link wall 19 and on the other side by a bar 20 which is interrupted when the capsules 1 pass through the rotating sealing rolls 16. The sealed capsules 21 are then conveyed to the collecting system 22 where they are ejected from the links 14.

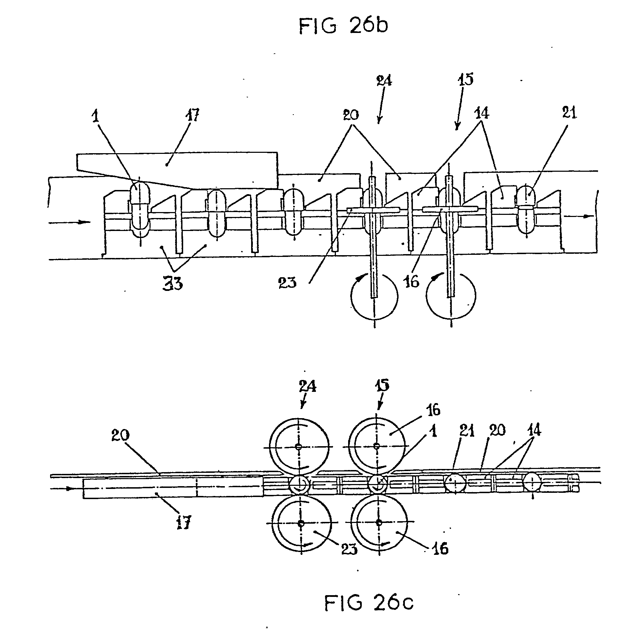

Figures 26b and 26c show an alternate embodiment to Figs. 25b and 25c wherein the capsules 1 are first conveyed through a wetting area 24 where they are contacted by rotating wetting rolls 23 (or alternatively with nozzles or jets) so as to precisely wet the cap side wall and/or the overlap of each capsule 1 with the sealing fluid.

The sealing of the capsule by hot metal device may be improved by further adding a softener to the mixture of water and an organic solvent. The softener may be selected from the following groups:

— poly-hydroxy-alcohols like glycerol, sorbitol, mannitol, and the like;

— dialkylphtalates preferably where alkyl is butyl;

— lower alkyl citrates wherein lower alkyl has 1—6 carbon atoms;

— polyglycols such as polyethyleneglycol and methoxy-propylene-glycol, and 1,2-propyleneglycol;

— esters of polyhydroxy-alcohols such as mono-, di- and tri-acetate of glycerol and the like;

— ricinoleic acid and esters thereof; and

— related materials and mixtures of the above.

The above softeners are used in a concentration range of about 0.1—10% based on the weight of the mixtures of water and organic solvents.

Alternatively, the above softeners could be added to gelatin used in the manufacture of the capsules in a concentration range of about 0.1 to 10% based on the weight of the gelatin.

In an alternative embodiment, the cap part side wall and/or the overlap may be contacted circumferentially with a normal steam of water. This can be accomplished by replacing the rotating wetting wheels 24 in Figs. 26b and 26c with steam nozzles so as to contact the steam over all of the circumference of the area to be sealed.

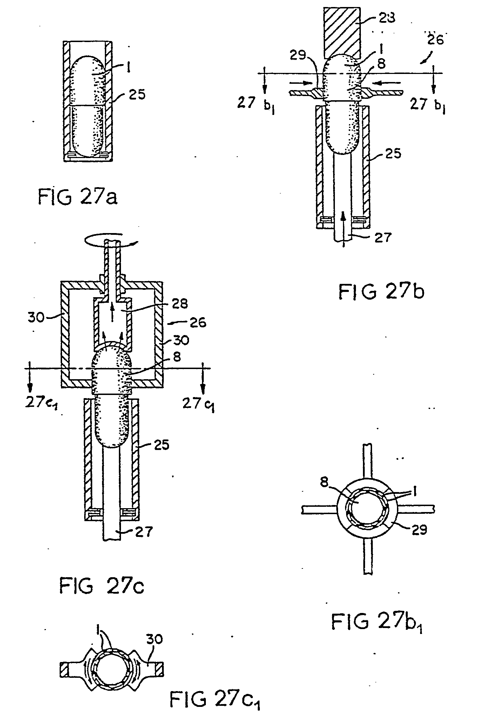

Figures 27a, b, c, b1, c1 show alternative embodiments. After having been filled and joined, the capsule 1 is held in a bushing 25 as in Fig. 27a, just before ejection from a capsule filling machine. A sealing device 26 can be fitted between the closing and ejection stations of a capsule filling machine. Fig. 27b illustrates one variant of a sealing device wherein the filled and joined capsule 1 is partially pushed out of the bushing 25 by a pusher 27; the cap 8 being partially introduced into a static holder 28. As shown in Fig. 27b and 27b$_1$, heated segments 29, as previously shown in Figs. 21 or 24, are applied against the outside of the cap wall 8 so as to hermetically seal the capsule 1. The static holder 28 assures a good holding of the capsule 1, thereby avoiding deformation during sealing.

Fig. 27c illustrates another embodiment of a sealing device wherein the filled and joined capsule 1 is partially pushed out of a bushing 25 by a pusher 27, the cap 8 being firmly maintained against a holder 28 by reduced pressure. Simultaneously, two teflon coated hot metal segments 30 are applied against the outside of the cap 8 at the overlap and alternatively rotated around the capsule 1, so as to achieve a 360° sealing. After sealing, the segments 30 are removed from the capsule 1, the reduced pressure is broken and the capsule 1 returns into the bushing 25 until ejection.

In one embodiment, the capsules are sealed by a 360° application of heat by metal devices, the temperature of which may be precisely adjusted. These devices may be wires, stamps, jaws, wheels or rolls with various dimensions and shapes, adapted to the particular seal shape and strength required. The heat may be applied by means of various combinations of movements:
— no rotation either of the heating device or of the capsule;
— rotation of the heating device around and against the capsule; or
— rotation of the capsule around and against the heating device.

The temperature of the heating devices may be in a range between about 70° and 200°C, preferably between 100° and 170°C.

The contact time may vary in a range between about 0.1 and 15 seconds, preferably between 0.5 to 3 seconds.

The presssure of the heating device against the cap side wall must be adjusted for the type of heating device, its relative movement with regard to the capsule, its temperature and the contact time.

As shown on Figs. 25, 26 and 27, the sealing may be done as a part of a high-speed two-piece capsule filling machine or connected to the exit of such a machine. The present invention may be used on an intermittently operating capsule filling machine, or on continuously operating filling machine.

When capsules contain low viscosity liquids, below 100 centipoises, the sealing is preferably performed capsules held in an upright position, preferably on the filling machine itself, thereby avoiding any leaking. Should the sealing be performed after random ejection from the filling machine, then some of the following are required to avoid leaking:
— sealing the capsules within a few seconds after ejection;
— cooling the liquid prior to filling into the capsules in order to increase the viscosity of the content; or
— adding a thickening agent to the liquid prior to the filling.

The hot metal devices can be surface treated, such as by chromium plating or titanium nitration coating in order to avoid sticking to the capsule during sealing, and to avoid unaesthetic surface alteration of the cap side wall (formation of wrinkles or corrugations) especially when the capsule is rotated against the sealing device. Other good surface coatings are teflon or silicone.

The following examples illustrate the invention:

Example 1

10,000 gelatin capsules size 2, filled with a lactose based placebo powder and closed, having a cap part wall containing 3,5% by weight of the dry capsule wall material of black iron oxide pigment (pharmaceutical grade pigment black II color index number 77499) and a natural transparent body part, were fed at a rate of 100,000 capsules per hour by means of a hopper to a paddle-wheel contacting station as shown in Fig. 10 where they were contacted during about one second with a mixture of 60% of pharmaceutical grade ethanol and 40% demineralized water by volume of the mixture, maintained at a temperature of about 18 to 23°C. Hereof the capsules were continuously fed via a chute by means of compressed air into the twin fluidized bed dryers part of the apparatus shown in Fig. 8 and Fig. 9 wherein each batch of capsules, collected in one dryer during a period of 6 minutes during which the mixture was first removed from the exposed surfaces of the capsules by a flow of air at 25°C, was then sealed by means of an air flow heated up to 55°C during 30 seconds and maintained at this temperature during 5 minutes, and was finally discharged from the dryer during a period of 30 seconds. The obtained capsules were completely sealed and could not be separated without visible mutilation.

11

Example 2

10,000 gelatin capsules size 2, natural transparent, were automatically filled with each 0.320 g of peanut oil (French Codex grade) and closed, at a rate of 11,000 capsules per hour and directly introduced in a random manner into the contacting station of the apparatus shown in Fig. 8 and Fig. 9 where they were in a first section contacted with a mixture of 50% of pharmaceutical grade ethanol and 50% of demineralized water (% by volume of total solution) at a temperature of between 15 to 20°C, during about 5 seconds and then the excess of the mixture was drained off by means of a compressed air blast at 20°C in the second section. Immediately thereafter, the capsules were continuously fed as in Example 1 into the same twin-fluidized bed dryers apparatus part as described in Example 1 where they were treated in the same manner except that the collection of capsules and removal of the mixture was with an air flow at 27°C during a period of 3 minutes and the sealing with an air flow heated up at 45°C during 15 seconds and maintained at this temperature during 2 minutes and 30 seconds before discharging. The obtained capsules were hermetically sealed. No leaking capsule was observed on a sample of 200 capsules stored for 3 months at 30°C.

Example 3

15,000 gelatin capsules size 0, filled with a lactose based placebo powder and closed, having a white opaque capsule wall containing 2%, by weight of dry material, of titanium dioxide pigment (pharmaceutical grade), and marked with two black logos printed with an alcohol soluble ink (pharmaceutical grade), were fed by means of a hopper at a rate of 90,000 capsules per hour into the contacting station shown in Fig. 3. In a first section of basket 10 contact was made with a mixture of 45% ethanol and 55% demineralized water (percents by volume of total solution) maintained at a temperature of about 10 to 14°C, during about 0.5 seconds; thereafter in a second section of basket 10 and in basket 11, part of the excess mixture was drained-off by means of a combination of an air blast at 20°C on top of the basket and an air suction device fitted near the bottom of the basket. Immediately thereafter, the capsules passed continuously within 5 to 7 seconds over a nail bed feeder (Modified Vibra-flow$^R$ model of Syntron-FMC Corporation, Homer, P.A., USA) characterized by about 9 nails by cm$^2$ whereby the rest of the excess of mixture was completely drained-off from the capsules and recycled into the tank 18 of Fig. 3.

Immediately thereafter, the capsules were continuously collected over a period of 3 minutes in a first rotating basket of a tumbler dryer as shown in Fig. 3 where their exposed outside surfaces were dried by a blast of air at 27°C. Immediately thereafter, the capsules were transferred into a second basket of the tumbler dryer where they were treated by an air blast at 42°C during 3 minutes while a new batch of capsules was collected and outside dried in the first basket, and before discharging from the tumbler dryer.

The obtained capsules were completely sealed and could not be separated without visible mutilation. The printed logos were not altered by the process. The same good result is obtained using an air blast at 60°C or 70°C (instead of 42°C) under the same conditions.

Example 4

10,000 gelatin capsules size 2, filled with a lactose based placebo powder, having a capsule wall containing 3.3%, by weight of dry material, (FD and C Red 3, a highly water-soluble dye), were fed by means of a hopper at a rate of 50,000 capsules per hour into a sealing apparatus identical to this of example 2 and treated in a same manner except that the ethanol/water mixture contained 93% of ethanol and 7% of water (percents by volume of total solution) that the contact time therewith was of 0.7 seconds at a temperature of between 17 and 19°C, and that the collection of capsules and removal of the mixture was with an air-flow at 32°C during 4 minutes and the sealing with an air flow heated up to 58°C during 30 seconds and maintained at this temperature during 2 minutes and 30 seconds before discharging. No significant amount of dye loss was noticed on the obtained capsules which were completely sealed and could not be separated without visible mutilation. During this trial the proportions of ethanol and water in the mixture were maintained at their constant pre-set value by continuous density control of the mixture and correlated adjustment thereof by addition of pure ethanol controlled by means of a regulator and a dosage pump.

Example 5

The sealed capsules of Examples 2 and 4 were tested for permeability to oxygen. The diffusion of oxygen therein was compared to the diffusion in identical unsealed reference gelatin capsules.

A small hole was made at one end of each capsule to allow a circulation of an oxygen free gas within the inside of the capsule. Outside of the capsule was room air (21% oxygen) with ambient relative humidity (55%) and temperature (23°C). The unsealed gelatin capsule samples were empty. The hermetically sealed capsule samples were carefully emptied under vacuum through the small hole before testing.

For each test sample, the mean values of the diffused oxygen from outside to within the capsule was as follows:

| Test sample | Nature of capsule | Quantity of content (in grams) | Diffusion of oxygen ($cm^3$/capsule/24 hours) |
|---|---|---|---|
| A) | sealed, size 2 gelatin capsule of Example 2 | 0.32 | 0.022 |
| B) | as in A), but unsealed | 0.32 | 2.8 |
| C) | sealed, size 2 gelatin capsule of Example 4 | 0.29 | 0.010 |
| D) | as in C), but unsealed | 0.29 | 2.5 |

It should be noted that the hermetic sealing of capsules against oxygen diffusion is also effective against diffusion of moisture vapor or other gases.

Example 6
10,000 gelatin capsules sealed as in Example 2 except that they were filled with pure DL-α-tocopheryl acetate maintained at a temperature of about 40°C.

Example 7
100 capsules, size 2, filled with a lactose based placebo powder, were contacted for 0.7 seconds with a mixture of 40% ethanol and 60% of water (percents by volume of total solution) and the excess of mixture was drained off. Both contacting and draining off steps were performed as in Example 2. The exposed outside surfaces of the capsules were then dried by an air flow at 58°C and 30% relative humidity for 1 minute.

A good bond of the overlap could also be obtained by applying the thermal energy locally to the overlapping sections of the capsule cap and body parts, at the outside of the cap part, by a metal stamp, coated with Teflon[R] at a temperature of 160°C during 0.3 seconds. The stamp treatment was either performed on one or more spots at the outside circumference of the overlapping sections of cap and body parts. All capsules could not be separated without visible mutilation. The visible mark left by the stamp made the capsules tamper evident.

Example 8
50 capsules, size 2, natural transparent, filled with peanut oil and closed, were oriented and held with the cap part upright in a holder which was then partly dipped, horizontally, into a mixture of 30% of ethanol and 70% of water (percents by volume of total solution as shown in Fig. 5) and such as the exposed body part and at least the edge of the cap of each capsule was contacted with the mixture during 1 second.

Thereafter, the excess of mixture was removed from the holder and the exposed outside surface of each capsule by means of an air flow at 25°C and 30% relative humidity. The capsules were then individually sealed by applying a hot metal wheel heated at 140°C during 1 second against the overlapping sections of capsule cap and body, at the cap outside, while simultaneously the capsule was submitted to a rotation of at least 360° (angular), thus resulting in a 360° hermetical seal which appeared at the cap outside as a continuous circumferential ring. No leaking was observed on a sample of 20 capsules stored for 3 months at 30°C and 60% relative humidity.

Example 9
200 gelatin capsules, size 2, natural transparent, filled with a lactose based placebo powder and closed were contacted with a mixture of 75% of ethanol and 25% of water (percents by volume of total solution) during 0.7 seconds and then the excess of mixture was drained off. Both contacting and draining off steps were performed as in Example 2. The capsules were then transferred onto a conveyor system with longitudinally juxtaposed, rotatably mounted metal rolls where they were axially and horizontally aligned, and simultaneously rotated and slowly conveyed. In a first section of the conveyor, the exposed outside surface of the capsules was dried by an air flow at room temperature and in a second section of the conveyor, the capsules were heated by an infra-red lamp for 6 minutes at a temperature of 58°C (measured on the rolls surface).

The obtained capsules were completely sealed and could not be separated without visible mutilation. The same good results are obtained when a sealing temperature of 70°C for 2 minutes is applied.

Example 10

200 gelatin capsules, size 2, natural transparent, filled with a lactose based placebo powder and closed, were treated as in Example 9, except that:

— The mixture composition was of 50% of ethanol and 50% of water (percents by volume of total solution).

— The capsules were sealed by being irradiated for 3 seconds with electromagnetic radiations (microwaves) at 2.4 GHZ at a field strength of 171 V/cm instead of using infra-red heating.

The obtained capsules were completely sealed and could not be separated without visible mutilation.

Example 11

600 gelatin capsules, size 2, natural transparent, filled with a lactose based placebo powder and closed were sealed as in Example 2 except that:

— 100 capsules were contacted, in the contacting step, with a mixture containing 90% of methanol and 10% of water and that the temperature was of 50°C in the sealing step.

— 100 capsules were contacted with a mixture containing 40% of methanol and 60% of water and that the temperature was of 40°C in the sealing step.

— 100 capsules were contacted with a mixture containing 80% of n-propanol and 20% of water and that the temperature was of 58°C in the sealing step.

— 100 capsules were contacted with a mixture containing 40% of n-propanol and 60% of water and that the temperature was of 50°C in the sealing step.

— 100 capsules were contacted with a mixture containing 70% of isopropanol and 30% of water and that the temperature was of 58°C in the sealing step.

— 100 capsules were contacted with a mixture containing 45% of L-propanol and 55% of water and that the temperature was of 50°C in the sealing step.

All 600 capsules were completely sealed and could not be separated without a visible mutilation.

All mixture compositions are given in percents by volume of the total solution.

Example 12

1900 two-piece gelatin capsules, size 2, filled with micronized lactose were introduced into a Uni-Glatt Laboratory Unit (available from Glatt GmbH, Binzen/Lörrach, West Germany) equipped with a Wurster type film coating insert (schematically shown in Fig. 15). The air flow having been adjusted (variable shutter opened at 60%) so as to have the capsules gently circulated within the apparatus, 200 ml of a mixture of 15% water (v/v) and 85% ethanol were intermittently sprayed onto the capsules, from the bottom of the device, during 3 seconds each 15 seconds. Pressure of the atomizing air in the spraying nozzle was 1 bar. The sealing fluid was fed to the nozzle by a peristaltic pump at a rate of 62 ml/minute. The temperature of the air was 25°C. After 16 minutes spraying and 3 minutes additional drying at this temperature, a sample of 100 capsules was taken off. The capsules were dry but unsealed or weakly sealed (only small spot seals), and could be reopened without visible damages to the capsule wall. For the 1800 remaining capsules the temperature of the air flow was raised within 4 minutes to 50°C, and the additional treatment time at this temperature was 10 minutes. The capsules obtained were perfectly sealed, 100% tamper-proof and without any visible alteration with regard to untreated references.

Example 13

1700 two-piece gelatin capsules, size 2, freshly filled with pasty lecithine (French Codex grade) were sealed in the same apparatus as in Examples 1 and 2, but the Wurster type film coating insert was replaced by a conventional fluidized bed spray-granulating insert, as shown in Fig. 14.

The air flow was adjusted so as to gently suspend and move the capsules. 250 ml of a mixture of 25% (v/v) water and 75% ethanol was intermittently sprayed from the top of the device onto the fluidized bed of capsules. The liquid was fed to the nozzle by a peristaltic pump at a rate of 125 ml/minute. Pressure of the atomizing air in the nozzle was 1 bar. The liquid could be sprayed each 30 secs during 15 secs without sticking of the capsules. Temperature of the air was 25°C.

After 6 minutes, the spray was stopped and the air was heated up to 50°C within 4 minutes. The capsules were maintained in the fluidized bed, at this temperature, during 5 minutes. The batch treatment time was 15 minutes.

The capsules obtained were completely sealed and leak-proof. After 3 months storage at 22°C and 50% relative humidity, no leakers could be detected.

Example 14

100 two-piece gelatin capsules, size 2, filled with lactose powder, were hermetically sealed on a modified capsule imprinting machine, as shown on Fig. 8.

The wetting rolls were made from a sponge-like polyurethane foam (the type felt 4bsr 80/10 supplied by Reticel, 54170 Colombey-les-Belles, France) and were continuously wetted with a mixture of 80% water, 20% ethylalcohol and 0.3% sodium laurylsulphate, based on the weight of the mixture. The wetting level of the rolls was controlled by transfer from a primary spongious roll rotating within a container containing the wetting mixture. The breadth of the rolls was adjusted to 3 mm so as to precisely wet the cap side wall and,

14

by capillary effect, the inside of the overlap of each capsule. The contact time of each capsule with the wetting rolls was 0.3 seconds.

After the wetting, the capsules are contacted with the sealing roll heated at 150°C during 0.3 seconds, with an apparatus as shown in Fig. 25, the sealing rolls were similar to those in Fig. 23.

The capsules obtained were hermetically sealed and could not be reopened without visible damage to the capsules.

Example 15

100 two-piece gelatin capsules, size 2, filled with lecithine (French Codex grade) were hermetically sealed as in Example 14, except that the wetting mixture had the following composition, by weight:

75% water
20% ethylacohol
5% glycerol.

The hermetically sealed capsules obtained were leakproof and were found to be about two times more resistant to an impact with an energy of 0.5 joules applied to the overlap on an impact testing equipment (supplied by Karl Frank GmbH, Weinheim-Birkenau, Western Germany) than reference capsules with the same filling material and sealed as in Example 14.

**Claims**

1. Method of sealing a hard shell gelatin capsule having coaxial cap and body parts which overlap when telescopically joined, the method comprising contacting the edge of the cap part of the capsule with a melting point depression sealing fluid to cause said fluid to be evenly distributed between the overlapping sections of the body and cap part and removing the excess sealing fluid from the exposed capsule surface of said capsule characterized in that

(a) the sealing fluid comprises from about 20% to about 98% of an aliphatic monohydric alcohol having one to four carbon atoms which may be substituted by one alkoxy group having one or two carbon atoms or mixtures thereof and from about 2% to about 80% of water; and

(b) whilst said sealing fluid is still present within the region of overlap thermal energy is applied to locally melt the contacting surfaces of the overlapping sections of the body and cap parts enabling a complete fusion together of said overlapping sections, wherein processes (i) to (iv)

(i) 75% ethanol/25% water as sealing fluid, and heat applied by hot air at 70°C;
(ii) 60% ethanol/40% water as sealing fluid, and heat applied by hot air at 70°C;
(iii) 60% ethanol/40% water as sealing fluid, and heat applied by infra-red heating means at 60°C;
(iv) 60% ethanol/40% water as sealing fluid, and heat applied by infra-red heating means at about 80°C are disclaimed.

2. A process according to claim 1, wherein heated air and/or infra-red energy are employed in step (b).

3. A process according to claim 1, wherein conduction energy derived from a hot metal stamp or bar which is heated to a temperature of 100°C to 170°C is used in step (b).

4. A process according to claim 1, wherein air heated to above 40°C is used in step (b).

5. A process according to claim 1 wherein the aliphatic monohydric alcohol is in the range of 30 to 95% and the water content is in the range of 5 to 70%.

6. A process according to claim 1, wherein the aliphatic monohydric alcohol is an ethanol.

7. A process according to anyone of the claims 1 to 6, wherein the heating step (b) is carried out in a fluidized bed.

8. Capsules sealed according to anyone of the claims 1 to 7.

9. An apparatus for sealing hard shell gelatin capsules, having coaxial cap and body parts which overlap when telescopically joined, characterized in that it has the following three working stations:

(A) a station in which at least the edge of each cap part is contacted with the melting point depression sealing fluid,

(B) a station for removing the mixture from the exposed capsule surface,

(C) a fluidized bed device to heat seal the overlapping sections of the cap and body parts of said capsules by effecting melting of the contacting surfaces.

**Patentansprüche**

1. Verfahren zur Versiegelung einer Gelatinekapsel mit harter Schale, mit koaxialen Kappen- und Körperteilen, welche bei teleskopischer Verbindung überlappen, welches Verfahren Kontaktieren der Kante des Kappenteiles der Kapsel mit einem den Schmelzpunkt senkenden Versiegelungsfluid, um die gleichmäßige Verteilung dieses Fluids zwischen den überlappenden Abschnitten des Körper- und Kappenteiles zu bewirken, und Entfernen des überschüssigen Versiegelungsfluids von der ausgesetzten Kapseloberfläche dieser Kapsel umfaßt, dadurch gekennzeichnet, daß

(a) das Versiegelungsfluid von etwa 20% bis etwa 98% eines aliphatischen einwertigen Alkohols mit 1 bis 4 Kohlenstoffatomen, welche durch eine Alkoxygruppe mit 1 bis 2 Kohlenstoffatomen substituiert sein koennen oder Mischungen davon, und von etwa 2% bis etwa 80% Wasser umfaßt; und

(b) während dieses Versiegelungsfluid noch innerhalb des Überlappungsbereichs anwesend ist, Wärmeenergie angewendet wird, um die kontaktierenden Oberflächen der überlappenden Abschnitte der Körper- und Kappenteile lokal zu schmelzen, wobei ein vollkommenes Miteinanderverschmelzen dieser überlappenden Abschnitte ermöglicht wird, wobei Verfahren (i) bis (iv)

(i) 75% Äthanol/25% Wasser als Versiegelungsfluid und Hitzeanwendung mittels Heißluft bei 70°C;

(ii) 60% Äthanol/40% Wasser als Versiegelungsfluid und Hitzeanwendung mittels Heißluft bei 70°C;

(iii) 60% Äthanol/40% Wasser als Versiegelungsfluid und Hitzeanwendung mittels Infrarotheizungsvorrichtung bei 60°C;

(iv) 60% Äthanol/40% Wasser als Versiegelungsfluid und Hitzeanwendung mittels Infrarotheizungsvorrichtung bei etwa 80°C ausgenommen (disclaimed) werden.

2. Verfahren nach Anspruch 1, worin erhitzte Luft und/oder Infrarot-Energie in Schritt (b) eingesetzt werden.

3. Verfahren nach Anspruch 1, worin Leitungsenergie, welche von einem heißen Metallstempel oder -stab stammt, welcher auf eine Temperatur von 100°C bis 170°C erhitzt wird, in Schritt (b) verwendet wird.

4. Verfhren nach Anspruch 1, worin auf über 40°C erhitzte Luft in Schritt (b) verwendet wird.

5. Verfahren nach Anspruch 1, worin der aliphatische einwertige Alkohol im Bereich von 30 bis 95% liegt, und der Wassergehalt im Bereich von 5 bis 70% liegt.

6. Verfahren nach Anspruch 1, worin der aliphatische einwertige Alkohol Äthanol ist.

7. Verfahren nach irgendeinem der Ansprüche 1 bis 6, worin der Erhitzungsschritt (b) in einem Fließbett durchgeführt wird.

8. Kapseln, welche nach irgendeinem der Ansprüche 1 bis 7 versiegelt sind.

9. Vorrichtung zur Versiegelung von Gelatinekapseln mit harter Schale, mit koaxialen Kappen- und Körperteilen, welche bei teleskopischer Verbindung überlappen, dadurch gekennzeichnet, daß sie die folgenden drei Arbeitsstationen aufweist:

(A) eine Station, in welcher zumindest die Kante jedes Kappenteiles mit dem den Schmelzpunkt senkenden Versiegelungsfluid in Kontakt gebracht wird,

(B) eine Station zum Entfernen der Mischung von der ausgesetzten Kapseloberfläche,

(C) eine Fließbettvorrichtung zum Hitzeversiegeln der überlappenden Abschnitte der Kappen- und Körperteile dieser Kapseln durch Bewirken des Schmelzens der kontaktierenden Oberflächen.

## Revendications

1. Procédé de scellement d'une capsule ou gélule en gélatine à enveloppe dure comportant un couvercle et un corps coaxiaux qui se recouvrent lorsqu'on les emboîte télecscopiquement, ce procédé consistant à mettre le bord du couvercle de la gélule au contact d'un fluide de scellement occasionnant une diminution du point de fusion et à répartir ce fluide de façon homogène entre les sections qui se recouvrent du corps et du couvercle et à éliminer le fluide de scellement en excès de la surface exposée de la gélule, caractérisé en ce que:

(a) le fluide de scellement comprend d'environ 20 à environ 98% d'un alcool monovalent aliphatique renfermant de 1 à 4 atomes de carbone, pouvant être substitué par un groupe alkoxy renfermant 1 ou 2 atomes de carbone, ou des mélanges de ceux-ci, et d'environ 2% à environ 80% d'eau; et

(b) ce fluide de scellement étant encore présent dans la région de recouvrement, on applique une énergie thermique pour fondre localement les surfaces des sections qui se recouvrent du corps et du couvercle, ceci permettant une fusion complète de ces zones de recouvrement, procédé dans lequel les étapes (i) à (iv) suivantes sont exclues:

(i) l'utilisation d'un mélange à 75% d'éthanol/25% d'eau comme fluide de scellement, et un chauffage à l'air chaud à 70°C;

(ii) l'utilisation d'un mélange à 60% d'éthanol/40% d'eau comme fluide de scellement, et un chauffage à l'air chaud à 70°C;

(iii) l'utilisation d'un mélange à 60% d'éthanol/40% d'eau comme fluide de scellement et l'application d'un chauffage à infrarouge à 60°C;

(iv) l'utilisation d'un mélange à 60% d'éthanol/40% d'eau comme fluide de scellement et un chauffage à infrarouge à environ 80°C.

2. Un procédé selon la revendication 1, dans lequel on emploie, dans l'étape (b), de l'air chauffé et/ou de l'énergie infrarouge.

3. Un procédé selon la revendication 1, dans lequel on utilise, dans l'étape (b), une énergie de conduction provenant d'un poinçon ou d'un barreau métallique chauffé à une température de 100°C à 170°C.

4. Un procédé selon la revendication 1, dans lequel, dans l'étape (b), on emploie de l'air chauffé à environ 40°C.

5. Un procédé selon la revendication 1, dans lequel l'alcool monovalent aliphatique est utilisé en concentration d'environ 30 à 95% et la teneur en eau est comprise entre 5 et 70%.

6. Un procédé selon la revendication 1, dans lequel l'alcool monovalent aliphatique est l'éthanol.

7. Un procédé selon l'une quelconque des revendications 1 à 6, dans lequel l'étape de chauffage (b) est mise en oeuvre dans un lit fluidisé.

**0 152 517**

8. Gélules scellées selon l'une quelconque des revendications 1 à 7.

9. Un dispositif de scellement de gélules en gélatine à enveloppe dure comportant un couvercle et un corps coaxiaux qui se recouvrent lorsqu'on les emboîte télescopiquement, caractérisé en ce qu'il comporte les trois postes de travail suivants:

(A) un poste dans lequel au moins le bord de chaque couvercle est mis au contact du fluide de scellement occasionnant une diminution du point de fusion;

(B) un poste d'élimination du mélange de la surface exposée de la gélule;

(C) un dispositif en lit fluidisé pour sceller à la chaleur les sections qui se recouvrent du couvercle et du corps de ces gélules par fusion des surfaces entrant en contact.

FIG 1

FIG 2

FIG 3

1

FIG 4

FIG 5

FIG 6

FIG 7

FIG 8

FIG 9

50

52,53

55

9

113
110
107
106
109
108

111
112
110

FIG 12

FIG 13

6

FIG 11

116

114

113

115

FIG 14

116

117

114

113

118

115

FIG 15

8

FIG 16

FIG 17

0 152 517

PROGRAMMATOR

AIR EXHAUST

FAN

SEALING
FLUID
TANK

P

DOSING
PUMP

SHUTOFF
VALVE

AIR FLOW
GAUGE

SEALING
FLUID
INLET

SEALING
APPARATUS

AIR INLET

REGULATOR

VARIABLE
SHUTTER

AIR
SUPPLY

FAN

HEAT
EXCHANGER

TEMPERATURE
CONTROL

PROGRAMMATOR

FIG 18

FIG 19a

FIG 19b

FIG 19c

FIG 20a

FIG 20b

FIG 20c

20a
20b
20c

3

1

2

2

2

2

FIG 20

FIG 21

# FIG 22a

# FIG 22b

# FIG 22c

FIG 23

FIG 24 a

FIG 24 b

FIG 24 c

FIG 25a

FIG 25b

FIG 25c

## FIG 26b

## FIG 26c

FIG 27a

FIG 27b

FIG 27c

FIG 27b₁

FIG 27c₁